(19) Europäisches Patentamt
European Patent Office
Office européen des brevets

(11) EP 2 217 222 B1

(12) **FASCICULE DE BREVET EUROPEEN**

(45) Date de publication et mention de la délivrance du brevet:
**17.02.2016 Bulletin 2016/07**

(21) Numéro de dépôt: **08864603.9**

(22) Date de dépôt: **03.12.2008**

(51) Int Cl.:
***A61K 9/70*** *(2006.01)* ***A61K 39/00*** *(2006.01)*

(86) Numéro de dépôt international:
**PCT/FR2008/052198**

(87) Numéro de publication internationale:
**WO 2009/080933 (02.07.2009 Gazette 2009/27)**

(54) **COMPOSITIONS POUR LA VACCINATION PAR VOIE CUTANÉE**

ZUSAMMENSETZUNGEN ZUR HAUTIMMUNISIERUNG

COMPOSITIONS FOR CUTANEOUS IMMUNISATION

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **03.12.2007 FR 0759505**

(43) Date de publication de la demande:
**18.08.2010 Bulletin 2010/33**

(73) Titulaire: **DBV Technologies**
**92100 Boulogne Billancourt (FR)**

(72) Inventeurs:
- **RONCO, Jorge**
**F-78120 Rambouillet (FR)**
- **GODEFROY, Sylvie**
**F-75015 Paris (FR)**
- **DUPONT, Bertrand**
**F-13100 Aix En Provence (FR)**

(74) Mandataire: **Becker, Philippe et al**
**Cabinet Becker & Associés**
**25, rue Louis Le Grand**
**75002 Paris (FR)**

(56) Documents cités:
**WO-A-02/071950** **WO-A-2004/030696**
**WO-A-2007/122226**

- **ANTHONI MINNA ET AL: "Smad3 signal transducer regulates skin inflammation and specific IgE response in murine model of atopic dermatitis." THE JOURNAL OF INVESTIGATIVE DERMATOLOGY AUG 2007, vol. 127, no. 8, août 2007 (2007-08), pages 1923-1929, XP002487281 ISSN: 1523-1747**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 217 222 B1

## Description

L'invention concerne le développement d'une méthode et d'un système de délivrance de vaccins par voie cutanée. L'invention concerne plus particulièrement l'utilisation d'un dispositif comportant un compartiment de condensation pour la vaccination epicutanée. L'invention concerne aussi des protocoles de vaccination épicutanée, permettant l'obtention d'une réponse immune efficace sans traitement de la peau. L'invention peut être mise en oeuvre chez tout mammifère, de préférence chez l'être humain, pour l'induction d'une réponse immune thérapeutique ou préventive contre tout type d'antigène.

La majorité des pathogènes pénètrent l'organisme à travers les muqueuses ou la peau. Ainsi la peau comme les muqueuses possèdent tous les outils nécessaires pour détecter les pathogènes et se défendre. Cette ligne de défense comprend notamment un contingent de cellules présentatrices d'antigène (CPA) professionnelles résidentes. Les seules CPA présentes dans l'épiderme en conditions normales sont les cellules de Langerhans (LC), des cellules dendritiques (DC) dérivées de la moelle osseuse. Après capture de l'antigène, les LCs activées migrent dans les ganglions lymphatiques drainant où elles initient une puissante réponse immune systémique. Ainsi, la peau représente une alternative potentielle au mode d'administration par injection traditionnel pour le développement de vaccins plus efficaces. De plus, le système immunitaire de la peau peut également être une cible idéale pour l'induction d'une tolérance dans différentes pathologies telles que les maladies auto-immunes, les allergies, et éventuellement le cancer.

Cependant, la couche cornée (CC), la couche la plus superficielle de la peau, est une barrière physicochimique efficace pour la pénétration de fluides, de larges molécules (polypeptides et protéines), de particules ou bien encore de microbes. Une variété de techniques ont ainsi été testées pour tenter d'obtenir une réponse immune efficace par voie épicutanée, c'est-à-dire par application d'un antigène sur la peau. Ces techniques impliquent toutes une altération physique de la couche cornée, par exemple par abrasion légère ou modérée (suite à l'application de bandes adhésives ou en utilisant du papier de verre), par application d'ultrasons ou de micro-aiguilles. Ces différentes techniques ont démontré une efficacité à des degrés divers, comme étape préalable à l'application de l'antigène, pour favoriser sa pénétration dans les couches superficielles de la peau (épiderme), contenant les cellules immunologiques, et l'induction d'une réponse immune. Ainsi, il a été démontré que la co-application d'un antigène et d'un adjuvant comme la toxine du cholera (CT) ou la toxine labile de *E. coli* (LT) sur une peau préalablement abrasée est efficace pour la génération d'une réponse immune anticorps (Glenn GM, Rao M, Matyas GR, Alving CR. Skin immunization made possible by cholera toxin. Nature. 1998. 391: 851 ; Glenn GM, Scharton-Kersten T, Vassell R, Matyas GR, Alving CR. Transcutaneous immunization with bacterial ADP-ribosylating exotoxins as antigens and adjuvants. Infect Immun. 1999. 67:1100-6.). En revanche, la simple hydratation de la peau, préalable à l'application d'un antigène, suivie ou non d'un traitement occlusif, n'a jamais donné lieu à une réponse anticorps spécifique. Celle-ci est obtenue uniquement dans le cas d'un traitement préalable de la peau conduisant à la perturbation physique de la couche cornée, ou de la co-administration de l'antigène avec un adjuvant puissant (CT ou LT).

D'autres travaux ont montré par la suite que l'utilisation d'un système occlusif pour maintenir une formulation vaccinale déposée sous forme liquide sur la peau d'animaux durant une période allant de 1 à 24 heures permettait d'induire une réponse immune chez l'animal et chez l'homme, mais uniquement après traitement de la peau. Cependant les réponses induites en utilisant un contact pour une période de 0 à 24 heures ne varient pas de façon qualitative et/ou quantitative.

Dans la demande WO 2004/030696 il a été envisagé d'induire une réaction immunitaire à l'aide d'une composition comprenant un antigène et un composé immunostimulateur, sous forme sèche, en utilisant un système PosIntro ou ISCOM.

L'utilisation de patchs dermiques pour obtenir une réaction immunitaire a également été proposée dans la demande internationale WO 2007/122226. Cependant, la méthode décrite dans ce document utilise un adjuvant.

Ainsi, sans traitement physique ou physicochimique préalable (papier de verre, éthanol, acétone, bande adhésive, glycérol), ou l'usage d'adjuvants puissants (LT ou CT) ou autre excipient facilitateur, aucune réponse anticorps spécifique de l'antigène n'est détectée.

La présente invention décrit pour la première fois une méthode permettant l'induction d'une réponse immune spécifique d'antigène par voie cutanée, sans qu'il soit nécessaire d'utiliser un adjuvant (CT ou LT) ou de prétraiter ou perforer la peau. Plus particulièrement, l'invention décrit une méthode de vaccination épicutanée, efficace même en l'absence d'adjuvant, qui repose sur l'utilisation d'une formulation vaccinale sèche appliquée sur la peau dans des conditions permettant la formation d'un compartiment de condensation. L'invention montre que la création et le maintien d'une hydratation de la peau induisent une perturbation de la CC qui permet le passage d'antigènes. Par ailleurs, l'eau accumulée sous le système de délivrance permet aussi à la formulation vaccinale sèche d'être réhydratée et solubilisée, et d'entrer en contact avec la peau. Nous avons montré dans des essais précliniques que l'invention permettait, contrairement aux différents systèmes cutanées décrits jusqu'à présent, d'induire une réponse immune anticorps spécifique de l'immunogène, sans co-administration d'adjuvant (de type CT ou LT) ni préparation (abrasion ou perforation) de la peau. De manière particulièrement remarquable, l'invention permet le transfert cutané de macro-molécules (principalement des polypeptides et protéines) sans aucun traitement actif, ce qui n'avait jamais été décrit dans l'état de l'art. Comme cela est illustré dans les exemples, une réponse immune particulièrement forte est observée après trois applications du vaccin selon le procédé de l'invention.

Un objet de l'invention réside ainsi dans l'utilisation d'une formulation vaccinale pour la préparation d'une composition pour la vaccination épicutanée d'un mammifère, de préférence un humain, caractérisée en ce que la formulation vaccinale est sous forme sèche et dépourvue d'adjuvant, et en ce qu'elle est administrée par application sur la peau du mammifère au moyen d'un dispositif cutanée formant au contact de la peau un compartiment de condensation possédant un volume compris entre 1 et 300 mm3/cm2 de surface du dispositif, ledit dispositif contenant une quantité de formulation vaccinale comprise entre 0,1 μg et 500 μg para cm2 de surface, le dispositif étant maintenu sur la peau pendant une période de 6 à 72 heures, et ladite application de la formulation sous forme sèche et dépourvue d'adjuvant sur la peau intacte induisant une réponse immune spécifique d'antigène carcatérisée par la production d'anticorps de type IgG.

La description divulgue également l'utilisation d'un dispositif d'administration cutanée pour la préparation d'une composition destinée à l'administration d'une formulation vaccinale comprenant un antigène, sans traitement préalable de la peau ni co-administration d'adjuvant, le dispositif d'administration cutanée contenant la formulation vaccinale sous forme sèche sans composé adjuvant, et formant au contact de la peau un compartiment de condensation contenant la formulation vaccinale, permettant ainsi la pénétration de l'antigène dans l'épiderme et l'induction d'une réponse immune en l'absence d'adjuvant ou traitement préalable de la peau.

La description divulgue également l'utilisation d'un dispositif d'administration cutanée pour l'administration d'une formulation vaccinale comprenant un antigène, sans traitement préalable de la peau ni co-administration d'adjuvant, le dispositif d'administration cutanée contenant la formulation vaccinale sous forme sèche sans composé adjuvant, et formant au contact de la peau un compartiment de condensation contenant la formulation vaccinale, permettant ainsi la pénétration de l'antigène dans l'épiderme et l'induction d'une réponse immune en l'absence d'adjuvant ou traitement préalable de la peau.

La description divulgue également une méthode pour induire une réponse immune spécifique d'antigène chez un mammifère, la méthode comprenant l'application sur la peau du mammifère, sans traitement préalable de la peau, d'un dispositif d'administration cutanée contenant une formulation vaccinale sous forme sèche comprenant l'antigène sans composé adjuvant, le dispositif formant au contact de la peau un compartiment de condensation contenant la formulation vaccinale, pendant une période de temps suffisante pour permettre la pénétration de l'antigène dans l'épiderme et l'induction d'une réponse immune en l'absence d'adjuvant ou de traitement préalable de la peau.

La description divulgue également une méthode pour induire par voie cutanée une réponse immune spécifique d'antigène, la méthode comprenant la création et le maintien d'une hydratation locale de la peau et la mise en contact de la peau hydratée avec une formulation vaccinale sèche dépourvue d'adjuvant, pendant une période de temps suffisante pour permettre la pénétration d'antigène et l'induction d'une réponse immune.

La description divulgue également une méthode pour induire une réponse immune systémique contre un antigène protéique chez un sujet, la méthode comprenant l'administration dudit antigène audit sujet par la voie épicutanée au moyen d'un dispositif cutané comprenant une paroi de fond (backing), la partie périphérique de ladite paroi de fond étant adaptée pour créer avec la peau une chambre hermétique, dans laquelle la paroi de fond maintient ledit antigène sur sa face en contact avec la peau, à l'intérieur de la chambre, ledit antigène étant enlevé de la paroi de fond après l'application du dispositif sur la peau et ensuite délivré au sujet par la voie épicutanée, ladite administration menant à l'induction d'une réponse immune systémique chez le sujet.

Dans un mode de mise en oeuvre particulier, la partie périphérique de la paroi de fond du dispositif cutané, possède des propriétés adhesives sur la peau humide.

Dans un autre mode de mise en oeuvre de l'invention, la réponse immune systémique induite après l'administration d'un antigène au moyen d'un dispositif cutané, comprend une réponse humorale.

Dans un mode de mise en oeuvre préféré, une condensation est formée à l'intérieur de la chambre suite à l'application du dispositif cutané sur la peau, ladite condensation causant ou augmentant le déplacement et la délivrance épicutanée de l'antigène. En particulier, cette condensation peut être formée en raison de transpiration.

La description divulgue également une méthode pour induire une réponse immune systémique contre un antigène protéique chez un sujet, dans laquelle ledit antigène est sous forme sèche ou sous forme de particules. Plus particulièrement, l'antigène peut être attaché au support sans adhésif au moyen des forces électrostatiques et/ou des forces de Van der Waals ou au moyen d'un revêtement adhésif sur le support.

L' antigène peut être appliqué sur le support au moyen d'un procédé de pulvérisation/séchage. Ledit antigène peut être également dissous ou dispersé dans un liquide, dans ce cas, il est maintenu sur le support dans un réservoir de matériel adsorbant.

La description divulgue également l'utilisation d'une formulation vaccinale sous forme sèche pour la préparation d'un médicament pour l'induction d'une réponse immune chez un mammifère, caractérisée en ce que la formulation vaccinale est appliquée au moyen d'un dispositif cutanée sans composé adjuvant sur la peau non traitée du mammifère, pendant une période de 48 heures environ.

La description divulgue également l'utilisation d'une formulation vaccinale sous forme sèche pour l'induction d'une réponse immune chez un mammifère, caractérisée en ce que la formulation vaccinale est appliquée au moyen d'un dispositif cutanée sans composé adjuvant sur la peau non traitée du mammifère, pendant une période de 48 heures environ.

La description divulgue également une méthode pour l'induction d'une réponse immune chez un mammifère, comprenant l'application d'une formulation vaccinale sous forme sèche au moyen d'un dispositif cutanée sans composé adjuvant sur la peau non traitée du mammifère, pendant une période de 48 heures environ.

La description divulgue également un dispositif cutané comprenant une paroi de fond (backing), la partie périphérique de ladite paroi de fond étant adaptée pour créer avec la peau une chambre hermétique, dans laquelle la paroi de fond maintient ledit antigène sur sa face en contact avec la peau, à l'intérieur de la chambre, ledit antigène étant enlevé de la paroi de fond après l'application du dispositif sur la peau et ensuite délivré au sujet par la voie épicutanée.

La description divulgue également une composition vaccinale, caractérisée en ce qu'elle comprend un dispositif d'administration cutanée formant un compartiment de condensation après application sur la peau d'un sujet, ledit compartiment comprenant une formulation vaccinale sous forme sèche et dépourvue de tout composé adjuvant.

L'invention résulte de la découverte inattendue qu'une réponse immune peut être induite par voie cutanée sur peau intacte (c'est-à-dire sans traitement perforant ou par abrasion physique et/ou chimique) et sans utilisation d'adjuvant. Cet effet a été obtenu en utilisant une formulation vaccinale sous forme sèche et en l'appliquant au moyen d'un dispositif formant un compartiment de condensation lorsqu'il est appliqué sur la peau et/ou pendant une période de temps adaptée.

Sans être tenu par un mécanisme d'action particulier, il est proposé que l'hydratation de la peau permette le gonflement des cornéocytes de la couche cornée, ainsi que l'intégration de molécules d'eau entre les domaines lipidiques des espaces intercellulaires. Cette hydratation induirait par ailleurs une perturbation de l'homéostasie cutanée, se traduisant entre autre par des modifications du profil des cytokines produites, des altérations morphologiques et phénotypique des cellules de Langerhans ou bien encore par l'augmentation du nombre de cellules mononucléées dans l'épiderme.

Malgré son rôle de barrière, il s'établit au travers de la peau entre l'organisme et l'atmosphère, et dans les conditions physiologiques, un flux d'eau dirigé vers l'atmosphère. Ce flux est connu sous le nom de perte insensible en eau (PIE). Le procédé de l'invention, qui met en oeuvre un compartiment de condensation, contribue à isoler une partie de la peau du milieu environnant. Au cours de ce phénomène, les échanges de vapeur d'eau entre le corps et l'atmosphère sont perturbés, ce qui aboutirait à l'accumulation d'eau dans le compartiment et aussi à une augmentation de l'hydratation de la peau, de tout l'épiderme et en particulier de la couche cornée (stratum corneum) qui constitue la principale barrière à l'absorption des substances. Nos résultats suggèrent que, chez *l'homme,* le flux d'eau très important mesuré après retrait du compartiment de condensation reflète l'évaporation de l'eau accumulée à la surface cutanée. Le compartiment de condensation du dispositif amène au fur et à mesure du temps à la saturation en vapeur d'eau (provenant de la PIE) du volume du compartiment, puis condensation, en arrivant à un état d'équilibre. En effet, au début de l'application, ce volume n'est pas saturé en vapeur d'eau, puisqu'il contient de l'air ambiant. Une fois la saturation de l'atmosphère atteinte, l'eau à l'état de vapeur pourrait alors se condenser sous forme d'eau liquide. Cette condensation serait alors alimentée par le flux d'eau résiduel en provenance de la peau. Dans ces conditions, un état d'équilibre est obtenu qui résulte dans l'accumulation de l'eau sous le dispositif étanche (voir Figure 1B).

L'invention permet ainsi de résoudre les inconvénients des méthodes de vaccination épicutanée de l'art antérieur, en proposant une méthode utilisable avec tout type d'antigène, compatible avec la production de dispositifs prêts à l'emploi, et ne nécessitant pas de traitement de la peau. La forte hydratation induite dans l'épiderme, avec accumulation d'eau dans et sur la surface du stratum corneum, permet de moduler le coefficient de partage entre les molécules de vaccin et la peau en présence d'eau, de gonfler les cornéocytes ou encore d'altérer l'organisation de la phase lipidique intercellulaire. Cette modification permet la dissolution du principe actif dans les liquides présents à la surface de la peau et son passage (par diffusion passive) au travers de la peau, sans qu'il soit nécessaire de perforer ou abraser la peau.

L'invention peut être mise en oeuvre avec tout dispositif d'administration cutanée formant un compartiment de condensation. Il s'agit de préférence de dispositifs de type patch, timbres ou pansements, de préférence de patchs. Le dispositif selon l'invention est typiquement de nature transdermique à diffusion passive, c'est-à-dire qu'il ne contient pas de moyens pour causer une perforation ou une abrasion de la peau. Il s'agit avantageusement d'un dispositif de type patch occlusif, comme par exemple un patch électrostatique produit selon la technologie VIASKIN$^R$ décrite dans la demande de brevet WO02/071950.

On désigne au sens de l'invention par compartiment l'espace laissé libre entre la peau et le fond (« backing ») du dispositif après application du dispositif sur la peau, ce backing n'étant pas collé sur la peau et laissant ainsi de la place pour l'accumulation d'une petite quantité d'eau sur la peau (voir par exemple les figures 1A et 1B). Les résultats obtenus montrent que l'utilisation d'un compartiment de condensation maintenant, pendant la durée de l'application, un certain degré d'hydratation due à l'expression naturelle de liquide par la peau en direction du compartiment, permet le passage d'antigènes macromoléculaires et l'induction d'une réponse spécifique. Ces résultats sont particulièrement surprenants compte tenu des données de l'art antérieur soulignant la nécessité de recourir à des adjuvants particuliers et à un traitement de la peau pour obtenir une réponse satisfaisante.

Dans l'invention, le dispositif est tel qu'il forme, au contact de la peau, un compartiment de condensation ayant un volume compris entre 1 et 300 mm3 par $cm^2$ de surface du dispositif. Un tel volume correspond à une hauteur de compartiment allant de 0,01 mm à 3 mm environ. Dans un mode plus préféré, le volume du compartiment est compris entre 2 et 200 mm3 par cm2 de surface du dispositif, encore plus préférentiellement entre 2 et 100 mm3 par cm2 de surface du dispositif, correspondant à une hauteur de 0,02 à 1 mm environ. La surface de diffusion sur la peau du dispositif peut être adaptée par l'homme du métier, et est typiquement comprise entre 0,2 et 5 $cm^2$, plus généralement entre 0,5 et 3 $cm^2$.

Il n'est pas nécessaire que les parois du compartiment de condensation (le backing du dispositif) soient totalement imperméables. Au contraire, une certaine perméabilité à la vapeur d'eau dans les conditions normales de température et de pression est acceptable, comme c'est le cas pour les polyéthylènes, dès lors que cette évaporation n'empêche pas le maintien d'une atmosphère saturée en vapeur d'eau, grâce à la présence d'eau liquide dans le compartiment.

Dans un mode particulier de mise en oeuvre, les parois du compartiment de condensation comprennent un matériau présentant un degré de perméabilité inférieur à 15 g/m2/24h, de préférence inférieur à 10 g/m2/24h, par exemple de l'ordre de 9 g/m2/24h, environ.

De tels matériaux peuvent être choisis par exemple parmi des polymères, copolymères, plastiques, céramiques, tout matériau biocompatible (par exemple le graphite), etc. Des exemples de polymères biocompatibles sont notamment le Polyéthylène (PE), Polycarbonate, polyester (PET), les plastiques cellulosiques (CA, CP), le chlorure de Polyvinyle (PVC), le polyméthylméthacrylate (PMMA), le polyuréthane (PUR), les Silicones (PTFE) ou le Polyfluorure de vinylidène (PVDF). Des exemples de copolymères biocompatibles sont notamment le copolymère d'Éthylène - Acétate de vinyle (EVA).

A titre d'examples de polymères présentant des degrés de perméabilité à la vapeur d'eau (MVTR, Moisture Vapor Transmission Rate) compatibles avec la présente invention, on peut citer notamment le CoTran® 9706 (MVTR = 26,4 $g/m^2/24H$), le CoTran® 9720 (MVTR = 9,4 $g/m^2/24H$) et le Coran® 9722 (MVTR = 7,9 $g/m^2/24H$).

Dans un mode de mise en oeuvre préféré, le compartiment de condensation est formé par un disque en polymère, par exemple en polyéthylène, de forme essentiellement circulaire (le backing) et de diamètre compris entre 2 et 100 mm environ, de préférence entre 5 et 70mm environ, qui est fixé (par exemple collé) sur un anneau de matériau biocompatible, par exemple en polyéthylène, présentant un diamètre extérieur essentiellement identique à celui du disque et un diamètre interne compris entre 1 et 50, de préférence entre 2 et 20 mm environ. L'épaisseur du disque et celle de l'anneau sont comprises par exemple entre 0,01 et 2 mm environ, ce qui définit un compartiment d'une hauteur comprise entre 0,01 et 2 mm, le volume du compartiment étant petit mais jamais nul, même si la flexibilité du backing détermine, en certains endroits un contact de ce dernier avec la peau. Le disque peut être fixé sur l'anneau par tout moyen adapté. Typiquement, l'anneau est adhésif ou rendu adhésif pour permettre sa fixation au disque.

Comme cela est illustré sur la Figure 1A, un prototype particulier de dispositif selon l'invention est composé par :

- un anneau [1] de matériel biocompatible, de diamètre variable et de hauteur de 0,01 à 2 mm environ, dépendant du volume du compartiment de condensation par cm2 de surface. Dans le mode représenté sur la figure 1A, l'anneau est adhésif sur sa face externe au moins, destinée à entrer en contact avec la peau.
- un disque [2] en matériel biocompatible, qui fait office de plafond du compartiment (conteneur du principe actif en forme sèche), collé sur l'anneau (par exemple à l'aide d'un adhésif). La surface du disque est typiquement de 5 mm de diamètre minimum. Le MVTR (Moisture Vapor Transmission) du disque (« backing ») est de préférence inférieur à 10g/m2/24h.
- une rondelle [3] de « release liner », protégeant la surface adhésive de l'anneau et destinée à être pelée avant application sur la peau. Cette rondelle est avantageusement un disque, qui recouvre le compartiment pour le fermer avant application du dispositif.

Le compartiment formé par le dispositif est un compartiment de condensation ayant des propriétés d'hydratation. Le pouvoir hydratant se traduit par la capacité à agir sur l'intégrité de l'épiderme sous le compartiment de condensation du dispositif induisant une augmentation du degré d'hydratation de l'épiderme, avantageusement dès la première heure d'application sur la peau, allant de 30% à 100% et plus précisément de 50% à 70%. De préférence, le pouvoir hydratant du compartiment de condensation doit être tel qu'il permet d'induire, à la surface externe de l'épiderme et dans le compartiment de condensation du dispositif, une augmentation de la quantité relative en eau allant de 40% à plus de 100% à partir de la première heure d'application, et plus préférentiellement, supérieure à 50%.

Dans un mode de mise en oeuvre préféré, le dispositif est maintenu sur la peau par des moyens assurant une étanchéité au compartiment de condensation. Tout moyen adhésif peut être utilisé à cet effet, y compris des matériaux de type gomme, polymère ou plastique ayant un fort pouvoir adhésif.

L'invention présente l'avantage de permettre la vaccination même sur peau intacte, c'est-à-dire une peau n'ayant pas subi de traitement particulier. Au sens de l'invention, on entend par traitement de la peau tout traitement abrasif ou perforant conduisant à une destruction au moins partielle de la couche superficielle au moins de la peau.

Par ailleurs, un autre avantage de l'invention est qu'elle permet d'obtenir une réponse immune importante, même sans co-administration d'adjuvant. Le terme adjuvant désigne au sens de l'invention tout composé qui exerce une action physico-chimique et/ou biologique sur la peau, facilitant la pénétration de l'antigène dans la peau, et qui augmente la réponse immunitaire contre l'antigène. Par action physico-chimique et/ou biologique, l'adjuvant tel que défini ici est un composé qui altère la perméabilité de l'épiderme par perturbation de la couche cornée, permettant ainsi le passage de l'antigène et son contact avec les cellules immunologiquement actives de l'épiderme. Des exemples de composés adjuvants sont notamment la toxine cholérique (CT) ou la LT (toxine thermolabile d'E-coli). D'autres exemples sont les transfersomes, décrits par Paul et al (Paul A, Cevc G, Bachhawat BK. Transdermal immunization with large proteins by ultradeformable drug carriers. Eur J Immunol. 1995, 25 (12) :3521-4), qui permettent de transporter un antigène dans les membranes.

L'invention peut être mise en oeuvre avec toute formulation vaccinale sous forme sèche. Le terme formulation vaccinale désigne toute formulation contenant des éléments antigéniques. Il s'agit typiquement d'une formulation comprenant un ou plusieurs antigènes, par exemple de nature peptidique, polypeptidique (ou protéique), lipidique et/ou saccharidique, ou de mélanges simples ou des conjugués chimiques de ces composés. Une formulation vaccinale préférée comprend une ou plusieurs molécules antigéniques : des molécules d'une masse moléculaire supérieure à 500 Da, plus souvent des polypeptides et des protéines supérieures à 1500 Da ayant une activité ou un but vaccinal préventif ou thérapeutique, ou des molécules d'une masse moléculaire supérieure à 500 Da destinées à une action locale (thérapeutique) sur l'épiderme. Typiquement, la formulation vaccinale comprend au moins un antigène de nature peptidique ou polypeptidique. Il peut s'agir par exemple d'antigènes bactériens, d'antigènes viraux, d'antigènes tumoraux, etc, comme par exemple un antigène du virus de la Grippe, un antigène d'un virus de l'Hépatite (/ou C), un antigène de *Haemophilus Influenzae,* un antigène du Virus respiratoire syncytial, un antigène de Salmonelle (Shigella), ou de la tuberculose (Tiphy), ou encore des antigènes caractéristiques de maladies auto-immunes, cancer, maladie d'Alzheimer, allergies, etc. La formulation vaccinale peut comprendre en outre tout excipient ou véhicule adapté à un usage pharmaceutique, de même que tout composé à activité immuno-modulatrice, comme par exemple une cytokine, un composé ligands des récepteurs Toll, etc.

La formulation vaccinale mise en oeuvre se présente sous « forme sèche », c'est-à-dire sous une forme deshydratée, et notamment une forme de poudre, particules, agglomérats, galette, forme séchée, etc. La forme sèche peut être obtenue par lyophilisation, ou par simple évaporation.

Dans un mode de mise en oeuvre particulier, la formulation vaccinale est sous forme de poudre.

Dans un autre mode de mise en oeuvre particulier, la formulation vaccinale est maintenue sur une surface du dispositif, typiquement sur le backing du compartiment, par des forces de nature chimique ou physicochimique, en l'absence de matériau adhésif sur cette surface. De préférence, la formulation vaccinale est maintenue sur une surface du dispositif, typiquement sur le backing du compartiment, par des forces électrostatiques ou des forces de Van der Waals.

Dans l'invention, le dispositif contient une quantité de formulation vaccinale comprise entre 0,1$\mu$g/cm2 et 500 $\mu$g/cm2, de préférence entre 20 $\mu$g et 300 $\mu$g par cm2, plus préférentiellement entre 20 $\mu$g et 100 $\mu$g par cm2. Il est entendu que cette quantité peut être adaptée par l'homme du métier en fonction des objectifs, de la pathologie, de la durée du traitement, du sujet, etc.

L'invention permet d'induire une réponse immune significative, même en l'absence d'adjuvant et de traitement de la peau. L'invention peut être utilisée pour la vaccination préventive ou thérapeutique contre tout type d'agent pathogène (virus, parasites, bactéries, protéines, etc.), contre des cellules tumorales ou contre des antigènes du soi, ou de traitement des individus allergiques. Les résultats présentés montrent l'induction d'une réponse spécifique d'antigène, notamment d'une réponse anticorps de type IgG.

L'amplitude de la réponse immune peut être adaptée en modifiant les temps de pose du dispositif, et/ou en procédant à des applications répétées. Comme cela est illustré dans les exemples, la réponse immune induite, après trois applications du vaccin de seulement 6 heures, est équivalente à celle observée pour une durée d'application de 24 heures (exemple 2). Nous avons ainsi démontré qu'une hydratation maintenue sur des périodes allant de 6 heures à 24 heures était suffisante pour développer une réponse immunitaire, même sans traitement préalable de la peau ni co-administration d'adjuvant.

Par ailleurs nous avons également prolongé le période d'application à 48 et à 72 heures (exemple 5). Les résultats obtenus montrent que le temps de 48 heures donne des résultats surprenants car la réponse anticorps spécifique augmente de presque un ordre de magnitude par rapport au temps de pose de 24 heures. Une application maintenue au-delà de 48 heures, par exemple un temps de pose de 72 heures, ne semble pas contribuer à augmenter davantage la réponse anticorps.

Dans un mode de mise en oeuvre préféré, l'invention comprend l'application successive au sujet de deux dispositifs au moins, de préférence trois au moins, chaque application étant espacée de 7 à 30 jours environ, de préférence de 10 à 20 jours, typiquement de 15 jours environ.

De préférence, pour chaque application, le dispositif est maintenu sur la peau pendant une période de 6 à 72 heures environ, de préférence de 6 à 48h environ, plus préférentiellement de 48 heures environ.

Un protocole particulièrement préféré pour l'induction d'une réponse immune comprend trois applications successives au moins d'un dispositif, pendant une période de 48 heures environ, chaque application étant espacée de 15 jours environ.

Dans ce qui précède, le terme «environ» désigne une variation de + ou - 10% par rapport à la valeur donnée.

L'invention montre aussi qu'une réponse anticorps importante est obtenue par vaccination épicutanée sur peau intacte lorsque la formulation vaccinale est sous forme sèche et est appliquée pendant une ou des périodes de 48h environ, de préférence pendant des périodes répétées de 48h environ.

L'invention est utilisable pour la vaccination contre tout antigène, chez tout mammifère. Elle peut être mise en oeuvre chez l'enfant ou l'adulte, est essentiellement non invasive, et ne nécessite pas l'emploi d'adjuvants.

D'autres aspects et avantages de la présente invention apparaîtront à la lecture des exemples qui suivent, qui doivent être considérés comme illustratifs et non limitatifs.

Légende des Figures

Figure 1 : **A** - Schéma du dispositif cutané avec compartiment de condensation: [1] anneau de matériel biocompatible, [2] disque du « backing » collé à l'aide d'un adhésif double face sur le disque, [3] rondelle de release liner à peler avant application protégeant l'adhésif. **B** - Schéma des flux de vapeur d'eau dans le compartiment de condensation.

Figure 2 : Induction d'une réponse immune anticorps spécifique de l'antigène par voie épicutanée sur peau intacte sans co-administration d'adjuvants en utilisant un dispositif contenant un compartiment de condensation.

Figure 3 : Induction d'une réponse immune anticorps anti-ovalbumine par voie épicutanée après 6 ou 24h d'application sur la peau d'un dispositif contenant un compartiment de condensation.

Figure 4 : Rapport entre la quantité de vapeur d'eau entrée dans le compartiment et la quantité d'eau condensée en fonction du temps. : ■: quantité d'eau accumulée dans le compartiment et ♦ quantité d'eau condensée dans le compartiment.

Quantité d'eau à l'état de vapeur après 20h.

Quantité de vapeur d'eau qui a condensé après 20h.

Figure 5 : Augmentation du pourcentage d'hydratation des différentes couches de l'épiderme (superficielle, superficielle et moyenne, épiderme entier) en fonction du temps d'application du dispositif contenant le compartiment de condensation.

Figure 6 : Augmentation de la quantité d'eau accumulée à la surface de la peau en fonction du temps d'application du dispositif contenant le compartiment de condensation.

Figure 7 : Potentialisation de la réponse immune anticorps anti-ovalbumine induite par voie épicutanée en prolongeant la durée d'application du dispositif contenant un compartiment de condensation sur la peau.

Figure 8 : Induction d'une réponse immune anticorps puissante spécifique de l'immunogène par voie épicutanée sans composé adjuvant ni traitement perforant ou abrasif de la peau en comparaison avec un système type patch.

Figure 9 : Induction d'une réponse immune anticorps anti-TT par voie épicutanée sans composé adjuvant avec de faibles doses d'antigène.

Figure 10 : Induction d'un épaississement de l'épiderme, relatif à une rupture de l'homéostasie cutanée, proportionnel au prolongement de la durée d'application du dispositif contenant un compartiment de condensation sur la peau.

Figure 11 : Induction de la pénétration de l'antigène dans la peau et de la prise en charge par les cellules dendritiques, proportionnels au prolongement de la durée d'application du dispositif contenant un compartiment de condensation sur la peau.

Figure 12 : Induction de la modification du réseau de cellules de Langerhans épidermiques, relatif à une activation de ces cellules, proportionnel au prolongement de la durée d'application du dispositif contenant un compartiment de condensation sur la peau.

Figure 13 : Induction de la production d'anticorps spécifique d'un antigène tumoral capables de cibler in vitro des cellules exprimant cet antigène après des applications de 24h sur la peau d'un dispositif contenant un compartiment de condensation chargé avec l'antigène tumoral.

Figure 14 : Induction d'une protection partielle contre une infection par le virus respiratoire syncytial (VRS) de souriceaux après une application de 48 heures sur la peau d'un dispositif contenant un compartiment de condensation chargé avec un antigène du VRS.

Exemples

**Exemple 1**

**Préparation d'un dispositif cutanée contenant un compartiment de condensation**

Des dispositifs d'application cutanée possédant un compartiment de condensation ont été préparés en utilisant comme « backing » un film de polyéthylène (Origine 3M), qui est recouvert par un anneau de mousse, qui donne la hauteur souhaitée au compartiment, rendu adhésif sur les deux faces (épaisseur = 0,7 mm). Ce dispositif présente ainsi une surface de diffusion sur la peau de 1 $cm^2$ et un compartiment d'expansion de l'air diffusé à travers la peau de 70 $mm^3$ (voir Figure 1A).

Plusieurs polymères présentant différentes perméabilités à la vapeur d'eau (MVTR, Moisture Vapor Transmission Rate) ont été utilisés, notamment le CoTran® 9706 (MVTR = 26,4 $g/m^2/24H$), le CoTran® 9720 (MVTR = 9,4 $g/m^2/24H$) et le CoTran® 9722 (MVTR = 7,9 $g/m^2/24H$).

**Exemple 2**

**Induction d'une réponse immune anticorps spécifique de l'antigène par voie épicutanée sans composé adjuvant ni traitement perforant ou abrasif de la peau**

Des dispositifs d'administration cutanée contenant un compartiment de condensation, préparés selon l'exemple 1 mais d'un volume de 10mm3/cm2, chargés avec un antigène modèle l'ovalbumine (OVA), la toxine tétanique (TT), un antigène tumoral ou un vaccin commercial contre la grippe, sous forme sèche, ont été appliqués durant 24 heures sur le dos de souris BALB/c femelles (n=6 à 10), tondu et épilé la veille des applications. Les applications ont été renouvelées 2 et 4 semaines après les premières. Les anticorps anti-antigènes de type IgG totales ont été dosés par ELISA dans les sérums des animaux prélevés 2 semaines après la dernière application. Les résultats présentés sur la figure 2 sont exprimés en titre anticorps spécifique de l'antigène. Il s'agit pour le sérum de chaque animal de l'inverse de la dilution à laquelle l'absorbance mesurée à 450 nm est égale au cut off déterminé à partir du sérum de souris naïves. Les résultats obtenus montrent l'induction d'une forte réponse immune spécifique d'antigène, et cela bien qu'aucun adjuvant n'ait été utilisé.

**Exemple 3**

**Induction d'une réponse immune anticorps anti-ovalbumine par voie épicutanée sans composé adjuvant après 6 ou 24h d'application sur la peau intacte**

Des dispositifs d'administration cutanée contenant un compartiment de condensation, préparés selon l'exemple 1 mais d'un volume de 10mm3/cm2, chargés avec l'OVA (100 $\mu$g) sous forme sèche, ont été appliqués durant 6 ou 24 heures sur le dos de souris BALB/c femelles (n=10), tondu et épilé la veille des applications. Les applications ont été renouvelées 2 et 4 semaines après les premières. Les anticorps anti-antigènes de type IgG totales ont été dosés par ELISA dans les sera des animaux prélevés 2 semaines après la dernière application. Les résultats, présentés sur la Figure 3, sont exprimés en titre anticorps spécifique de l'antigène. Il s'agit pour le sérum de chaque animal de l'inverse de la dilution à laquelle l'absorbance mesurée à 450 nm est égale au cut off déterminé à partir du sérum de souris naïves. Les résultats obtenus montrent l'induction d'une forte réponse immune spécifique d'antigène avec des temps de pose de 6 ou 24 heures.

**Exemple 4**

**Etude de la quantité et de l'état physique de d'eau disponible dans le compartiment de condensation**

La peau laisse s'évaporer une quantité physiologique de vapeur d'eau. Cette évaporation est exprimée par la PIE. Elle provoque une augmentation de la quantité de vapeur d'eau dans le compartiment de condensation. L'augmentation de la quantité de vapeur provoque à son tour une augmentation de la pression partielle de vapeur d'eau dans le compartiment. A partir d'une pression de 53,75 mbar à 35°C, le compartiment est en condition saturante. Au delà de cette pression, la vapeur se condense en eau liquide qui se dépose à la surface de la peau et peut remplir le compartiment de condensation. Par ailleurs, dans la composition du compartiment de condensation, le polymère du backing utilisé dans cet exemple n'est pas totalement imperméable et laisse s'échapper une certaine quantité de vapeur définie par sa perméabilité à la vapeur d'eau (Moisture Vapor Transmission Rate, MVTR).

Ainsi, dans ce système, il existe un équilibre entre d'une part la quantité d'eau qui entre dans le compartiment sous forme de vapeur d'eau et celle qui en sort soit par transmission au travers du film polymère, soit par condensation. Ces différentes quantités ont été évaluées de la manière suivante :

$$Q_1 = PIE*S*t$$

$$Q_2 = MVTR*S'*t$$

$$Q = Q_1 - Q_2$$

$$P = nRT/V$$

Avec $Q_1$ = masse de vapeur à chaque temps qui entre dans le compartiment ; $Q_2$ = quantité de vapeur d'eau qui s'est échappé du polymère ; Q = quantité de vapeur d'eau restant dans le compartiment ; P = pression dans le compartiment ; S = surface de libération de la vapeur d'eau à travers la peau ; S' = surface de libération de la vapeur d'eau à travers le polymère ; t = le temps.

Les quantités d'eau avant et après 20 heures d'application du compartiment de condensation ont été évaluées par des mesures de PIE sur des peaux prélevées des dos de rats hairless. Les déterminations ont été faites en comparaison avec les zones de la peau non soumisses au dispositif. Les fragments de peau ont été montés sur des cellules de diffusion de Franz. Dans le compartiment receveur, un tampon PBS pH = 7,4 permet de conserver la peau dans un état d'hydratation comparable à l'état physiologique. Quand les cellules de Franz sont prêtes, elles sont placées dans un bain marie à 37°C et conservées ainsi pendant deux heures avant toutes autres manipulations, de manière à équilibrer la température à celle des flux physiologiques.

Les résultats, présentés sur la figure 4, montrent que l'eau introduite sous forme de vapeur dans le compartiment commence à se condenser assez rapidement après l'application du dispositif. D'après ces résultats, qui expriment les données initiales et finales du système, les quantités de vapeur d'eau et d'eau liquide calculées au bout de 20 heures sont, respectivement, de 1,08 mg/cm2 d'eau liquide et de 0,3 mg/cm2 sous forme de vapeur d'eau. D'après l'examen visuel au cours de l'expérience l'eau paraît s'accumuler sous forme liquide à la surface de la peau.

**Exemple 5**

**Augmentation du pourcentage d'hydratation des différentes couches de l'épiderme (superficielle, superficielle et moyenne, épiderme entier) au cours du temps après application du dispositif à compartiment de condensation**

L'Hydrascan® est un appareil de mesure de l'hydratation de l'épiderme mis au point par la société Dermscan®. Le principe des mesures est basé sur le transfert thermique de passage (TTT) : une pulsation de chaleur constante est générée par un stimulateur proche du détecteur thermique et est propagée à travers l'épiderme. Chaque changement de température dans la peau est proportionnel à la quantité d'eau dans la peau (Girard P., Beraud A. and Sirvent A. - Study of three complementary techniques for measuring cutaneous hydration in vivo in human subjects: NMR spectroscopy, transient thermal transfer and corneometry - application to xerotic skin and cosmetics. - Skin Research and Technology, 2000, 6: 205-213).

Les mesures sont données en mW/°C (c'est la conductance thermique, qui mesure la faculté de conduire la chaleur entre deux points, en fonction de la différence de t° entre ces deux points).

L'appareil permet de mesurer l'hydratation dans l'épiderme en distinguant 3 couches : la couche superficielle, la couche intermédiaire et la couche profonde de l'épiderme.

Cette étude a été réalisée sur un groupe de 15 volontaires : femmes 18-50 ans, caucasiennes, peau normale, dispositif avec compartiment de condensation de 70 mm3 appliqué sur l'avant bras à 28°C. La cinétique d'hydratation a été effectuée avec des mesures d'Hydrascan à temps : 0H, 1H, 2H, 3H et 4H. Les déterminations d'hydratation de l'épiderme sous le compartiment de condensation ont été réalisées à chaque temps en comparaison avec une détermination sur une zone de la peau non soumisse à l'application du compartiment de condensation. Les résultats obtenus sont présentés sur la Figure 5 et montrent une augmentation du degré d'hydratation dans le compartiment de condensation, dès la première heure d'application, hydratation qui peut être maintenue au cours du temps.

**Exemple 6**

**Augmentation de l'hydratation à la surface de la peau au cours du temps après l'application du dispositif à compartiment de condensation**

Cette étude a été réalisée sur un groupe de 15 volontaires : femmes 18-50 ans, caucasiennes, peau normale, dispositif avec compartiment de condensation de 70 mm3 appliqué sur l'avant bras à 26°C. La détermination de la perte insensible en eau (PIE) a été effectuée avec un Tewameter® 210 (Courage and Khazaka Electronic Gmbh., Köln, Germany).

La détermination de la quantité d'eau accumulée sous le compartiment de condensation, donc sur la peau, a été calculée par des mesures de PIE enregistrées chaque seconde pendant 90 secondes une fois que le dispositif a été enlevé. Les aires sous la courbe ont été calculées pour chaque temps de mesure. Les mesures ont été réalisées à 0h 1h, 2h, 3h et 4h après l'application du compartiment de condensation.

Les résultats obtenus sont présentés sur la Figure 6 et montrent une augmentation de la quantité d'eau accumulée à la surface de la peau au cours du traitement.

**Exemple 7**

**Potentialisation de la réponse immune anticorps anti-ovalbumine induite par voie épicutanée en prolongeant la durée d'application sur la peau**

Des dispositifs contenant un compartiment de condensation, préparés selon l'exemple 1 mais d'un volume de 10mm3/cm2, chargés avec l'OVA (100 μg) ou la TT (100 μg) sous forme sèche, ont été appliqués durant 24 ou 48 heures sur le dos de souris BALB/c femelles (n=6 pour la figure 7A, n=10 pour la figure 7B), tondu et épilé la veille des applications. Les applications ont été renouvelées selon deux régimes différents :-Protocole 1 : 2 et 4 semaines après les premières (figure 7A et 7B), - Protocole 2 : 2, 7, 9, 14, 21 et 28 jours après les premières (figure 7B).

Les anticorps anti-OVA de type IgG totales ont été dosés par ELISA dans les sera des animaux prélevés 2 semaines après la dernière application. Les résultats, présentés sur la figure 7A et 7B, sont exprimés en titre anticorps spécifique de l'antigène. Il s'agit pour le sérum de chaque animal de l'inverse de la dilution à laquelle l'absorbance mesurée à 450 nm est égale au cut off déterminé à partir du sérum de souris naïves. Les résultats montrent une réponse anticorps spécifique particulièrement prononcée pour des temps de pose de 48 heures.

**Exemple 8**

**Induction d'une réponse immune anticorps puissante spécifique de l'immunogène par voie épicutanée sans composé adjuvant ni traitement perforant ou abrasif de la peau en comparaison avec un système type patch**

Des dispositifs d'administration cutanée contenant un compartiment de condensation, préparés selon l'exemple 1 mais d'un volume de 10mm3/cm2, chargés avec un immunogène modèle la toxine cholérique (CT), sous forme sèche, ont été appliqués durant 24 heures sur le dos de souris BALB/c femelles (n= 10), tondu et épilé la veille des applications. Les applications ont été renouvelées à 2 et 4 semaines après les premières. Par ailleurs, des systèmes type patch composé d'une gaze sur laquelle l'immunogène est adsorbée sous forme liquide, recouverte d'un sparadrap ordinaire ont été appliqués sur le dos de souris BALB/c femelles dans les même conditions que le dispositif décrit précédemment. Les anticorps anti-antigènes de type IgG totales ont été dosés par ELISA dans les sérums des animaux prélevés 2 semaines après la dernière application. Les résultats présentés sur la figure 8 sont exprimés en titre anticorps spécifique de l'antigène. Il s'agit pour le sérum de chaque animal de l'inverse de la dilution à laquelle l'absorbance mesurée à 450 nm est égale au cut off déterminé à partir du sérum de souris naïves.

Les résultats montrent l'induction d'une plus forte réponse immune spécifique de l'immunogène lorsque le dispositif d'administration cutanée contenant un compartiment de condensation était utilisé, en comparaison avec le système type patch.

**Exemple 9**

**Induction d'une réponse immune anticorps anti-TT par voie épicutanée sans composé adjuvant pour de faible dose d'antigène**

Des dispositifs d'administration cutanée contenant un compartiment de condensation, préparés selon l'exemple 1 mais d'un volume de 10mm3/cm2, chargés avec la TT (10 ou 100 μg) sous forme sèche, ont été appliqués durant 24 ou 48 heures sur le dos de souris BALB/c femelles (n=10), tondu et épilé la veille des applications. Les applications ont été renouvelées 2 et 4 semaines après les premières. Les anticorps anti-antigènes de type IgG totales ont été dosés par ELISA dans les sera des animaux prélevés 2 semaines après la dernière application. Les résultats, présentés sur la Figure 9, sont exprimés en titre anticorps spécifique de l'antigène. Il s'agit pour le sérum de chaque animal de l'inverse de la dilution à laquelle l'absorbance mesurée à 450 nm est égale au cut off déterminé à partir du sérum de souris naïves.

Les résultats montrent l'induction de titre anticorps spécifique d'antigène équivalent pour des dispositifs d'administration cutanée contenant un compartiment de condensation chargés avec <u>100 μg et appliqués 24 heures</u>, et pour des dispositifs d'administration cutanée contenant un compartiment de condensation chargés avec <u>10 μg et appliqués 48 heures.</u>

**Exemple 10**

**Induction d'un épaississement de l'épiderme proportionnel au prolongement de la durée d'application du dispositif contenant un compartiment de condensation sur la peau**

Des dispositifs d'administration cutanée contenant un compartiment de condensation, préparés selon l'exemple 1 mais d'un volume de 10mm3/cm2, chargés avec l'OVA (100 μg/cm2) sous forme sèche, ont été appliqués durant 0, 24 ou 48 heures sur le dos de souris BALB/c femelles (n= 8 à 16), tondu et épilé la veille des applications. La zone de peau sous le compartiment de condensation a été prélevée et congelés. Les coupes de peau obtenues à partir des échantillons ont été colorées avec Hemalun-eosin. Les résultats, présentés sur la Figure 10, sont représentatifs des observations faites au microscope au grossissement x200.

Les résultats montrent que l'application du dispositif d'administration cutanée contenant un compartiment de condensation chargé avec l'OVA induit un épaississement de l'épiderme, visible après 24 heures d'application et majeur après 48 heures d'application.

**Exemple 11**

**Induction de la pénétration de l'antigène dans la peau et de la prise en charge par les cellules dendritiques, proportionnels au prolongement de la durée d'application du dispositif contenant un compartiment de condensation sur la peau**

**[0082]** - Des dispositifs d'administration cutanée contenant un compartiment de condensation, préparés selon l'exemple 1 mais d'un volume de 10mm3/cm2, chargés avec l'OVA (100 μg/cm2) couplé avec l'Alexa 488 ($A_{488}$) sous forme sèche, ont été appliqués durant 6, 24 ou 48 heures sur le dos de souris BALB/c femelles (n= 10 à 20), tondu et épilé la veille des applications. La zone de peau sous le compartiment de condensation a été prélevée. Après séparation de l'épiderme du derme (trypsine 0.5%), les cellules épidermiques ont été isolées par resuspensation mécanique et les cellules du derme par digestion enzymatique (collagénase/DNase). L'OVA-$A_{488}$ a été détectée dans les suspensions cellulaires et parmi les cellules dendritiques, cellules identifiées grâce au marqueur CD11c, par cytométrie de flux (FACscallibur et logiciel Cell quest). Les résultats présentés sur la figure 11, sont exprimés en pourcentage de cellules fluorescentes (OVA-$A_{488}^+$) parmi les cellules totales et parmi les cellules dendritiques ($CD11c^+$) de chaque suspension cellulaires (épiderme et derme).

Les résultats montrent que des cellules épidermiques et dermiques sont OVA-$A_{488}^+$ et que leur proportion augmente avec la prolongation de la durée d'application du dispositif d'administration cutanée contenant un compartiment de condensation chargé avec OVA-$A_{488}$. Une partie de ces cellules OVA-$A_{488}^+$ sont des cellules dendritiques (CD11c+) et de la même manière, le pourcentage de cellules dendritiques OVA-$A_{488}^+$ augmente avec la prolongation de la durée d'application du dispositif d'administration cutanée contenant un compartiment de condensation.

**[0084]** - Des dispositifs d'administration cutanée contenant un compartiment de condensation, préparés selon l'exemple 1 mais d'un volume de 10mm3/cm2, chargés avec l'OVA (100 μg/cm2) couplé avec l'Alexa 488 (A488) sous forme sèche, ont été appliqués durant 24 heures sur le dos de porc (n= 1), tondu et épilé la veille des applications.

La zone de peau sous le compartiment de condensation a été prélevée. Après séparation de l'épiderme du derme (trypsine 0.5%), les cellules épidermiques ont été isolées par resuspensation mécanique et les cellules du derme par digestion enzymatique (collagénase/DNase). L'OVA-$A_{488}$ a été détectée dans les suspensions cellulaires et parmi les cellules dendritiques, cellules identifiées grâce au marqueur SLA-II, par cytométrie de flux (FACscallibur et logiciel Cell quest). Les résultats présentés sur la figure 11, sont exprimés en pourcentage de cellules fluorescentes (OVA-$A_{488}^+$) parmi les cellules totales et parmi les cellules dendritiques ($SLA\text{-}II^+$) de chaque suspension cellulaires (épiderme et derme).

Les résultats montrent que des cellules épidermiques et dermiques sont OVA-$A_{488}^+$ après une application de 24 heures du dispositif d'administration cutanée contenant un compartiment de condensation chargé avec OVA-$A_{488}$. Une partie de ces cellules OVA-$A_{488}^+$ sont des cellules dendritiques (SLA-II+).

Les résultats de la figure 11 montrent que le dispositif d'administration cutanée contenant un compartiment de condensation favorise la pénétration de l'antigène dans la peau et sa prise en charge par les cellules dendritiques.

**Exemple 12**

**Induction de la modification du réseau de cellules de Langerhans épidermiques, proportionnel au prolongement de la durée d'application du dispositif contenant un compartiment de condensation sur la peau**

Des dispositifs d'administration cutanée contenant un compartiment de condensation, préparé selon l'exemple 1 mais d'un volume de 10mm3/cm2, chargés avec l'OVA (100 μg) sous forme sèche, ont été appliqués durant 0, 24 ou 48 heures sur le dos de souris BALB/c femelles (n= 5), tondu et épilé la veille des applications. La zone de peau sous le compartiment de condensation a été prélevée et congelée. Les cellules de Langherans ont été identifiées sur les coupes de peau obtenues à partir des échantillons grâce au marqueur CD207. Les résultats, présentés sur la Figure 12, sont représentatifs des observations faites au microscope au grossissement x200.

Les résultats montrent que l'application du dispositif d'administration cutanée contenant un compartiment de condensation chargé avec l'OVA induit une modification du réseau de cellules de Langherans de l'épiderme, visible après 24 heures d'application et majeur après 48 heures d'application. Ce phénomène est caractéristique d'une activation de ces cellules.

**Exemple 13**

**Induction de la production d'anticorps spécifique d'un antigène tumoral capables de cibler *in vitro* des cellules exprimant cet antigène après des applications de 24h sur la peau d'un dispositif contenant un compartiment de condensation chargé avec l'antigène tumoral**

Des dispositifs d'administration cutanée contenant un compartiment de condensation, préparés selon l'exemple 1 mais d'un volume de 10mm3/cm2, chargés avec un antigène tumoral sous forme sèche, ont été appliqués durant 24 heures sur le dos de souris BALB/c femelles (n=6), tondu et épilé la veille des applications. Les applications ont été renouvelées 2 et 4 semaines après les premières. Les anticorps anti-antigènes de type IgG totales ont été dosés par ELISA dans les sera des animaux prélevés 2 semaines après la dernière application. Les sera des animaux ont été incubés avec des cellules exprimant l'antigène tumoral. La fixation des anticorps anti-antigène tumoral sur les cellules a été détectée après marquage avec un anticorps anti-Ig de souris fluorescent par cytométrie de flux (FACscallibur et logiciel Cell quest). Les résultats figure 13 sont exprimés sous forme d'histogramme présentant l'intensité de fluorescence en fonction du nombre de cellules.

Les résultats montrent que les anticorps anti-antigène tumorale contenu dans le sérum de souris immunisées par voie épicutanée en utilisant un dispositif d'administration cutanée contenant un compartiment de condensation chargé avec l'antigène tumoral, reconnaissent *in vitro* des cellules exprimant l'antigène tumoral.

**Exemple 14**

**Induction d'une protection partielle contre une infection par le virus respiratoire syncytial (VRS) de souriceaux après une application de 48 heures sur la peau d'un dispositif contenant un compartiment de condensation chargé avec un antigène du VRS**

Des dispositifs d'administration cutanée contenant un compartiment de condensation, préparés selon l'exemple 1 mais d'un volume de 10mm3/cm2, chargés avec un antigène du virus respiratoire syncytial sous forme sèche, ont été appliqués durant 48 heures sur le dos de souriceaux BALB/c (7 jours). L'antigène a été co-administré avec un adjuvant par voie intra-nasale en contrôle. L'épreuve virale est réalisée 23 jours après la vaccination par voie nasale. Le dosage des ARN viraux a été réalisé à partir des poumons prélevés 5 jours après infection. Les résultats présentés figure 14 sont exprimés en quantités relative d'ARN viraux dans les poumons par rapport à un gène de référence et normalisé par rapport à un témoin positif (individu non vacciné).

Les résultats montrent que la charge virale des souriceaux après une application de 48 heures sur la peau d'un dispositif contenant un compartiment de condensation chargé avec un antigène du VRS est diminuée de moitié par rapport au souriceaux non traités.

**Revendications**

1. Utilisation d'une formulation vaccinale pour la préparation d'une composition pour la vaccination épicutanée d'un mammifère, de préférence un humain, **caractérisée en ce que** la formulation vaccinale est sous forme sèche et dépourvue d'adjuvant, et **en ce qu'**elle est administrée par application sur la peau intacte du mammifère au moyen d'un dispositif cutanée formant au contact de la peau un compartiment de condensation possédant un volume compris entre 1 et 300 mm3/cm2 de surface du dispositif, ledit dispositif contenant une quantité de formulation vaccinale comprise entre 0,1 µg et 500 µg par cm2 de surface, le dispositif étant maintenu sur la peau pendant une période de 6 à 72 heures, et ladite application de la formulation sous forme sèche et dépourvue d'adjuvant sur la peau intacte induisant une réponse immune spécifique d'antigène **caractérisée par** la production d'anticorps de type IgG.

2. Utilisation selon la revendication 1, **caractérisée en ce que** les parois du compartiment de condensation comprennent un matériau présentant un degré de perméabilité inférieur à 10 g/m2/24h.

3. Utilisation selon l'une quelconque des revendications précédentes, **caractérisée en ce que** le compartiment de condensation possède un volume compris entre 2 et 100 mm3 par cm2 de surface.

4. Utilisation selon l'une quelconque des revendications précédentes, **caractérisée en ce que** le compartiment de condensation permet d'induire une augmentation du degré d'hydratation dans le compartiment de condensation et à la surface externe de l'épiderme de 50% au moins après une heure d'application.

5. Utilisation selon l'une quelconque des revendications précédentes, **caractérisée en ce que** le dispositif est maintenu sur la peau par des moyens adhésifs.

6. Utilisation selon l'une quelconque des revendications précédentes, **caractérisée en ce que** la formulation vaccinale est sous forme de poudre.

7. Utilisation selon l'une quelconque des revendications précédentes, **caractérisée en ce que** la formulation vaccinale est maintenue sur une surface du dispositif par des forces de nature chimique ou physicochimique, en l'absence de matériau adhésif sur cette surface.

8. Utilisation selon l'une quelconque des revendications précédentes, **caractérisée en ce que** la formulation vaccinale est maintenue sur une surface du dispositif par des forces électrostatiques ou des forces de Van der Waals.

9. Utilisation selon l'une quelconque des revendications précédentes, **caractérisée en ce que** le dispositif contient une quantité de formulation vaccinale comprise entre 20 µg et 300 µg par cm2 de surface, plus préférentiellement entre 20 µg et 100 µg par cm2 de surface.

**10.** Utilisation selon l'une quelconque des revendications précédentes, **caractérisée en ce que** la formulation vaccinale comprend au moins un antigène de nature peptidique ou polypeptidique ou des conjugués polypeptidique avec des composants de différente nature chimique.

**11.** Utilisation selon l'une quelconque des revendications précédentes, **caractérisée en ce qu'**elle comprend l'application successive au sujet de deux dispositifs au moins, de préférence trois au moins, chaque application étant espacée de 7 à 30 jours environ, de préférence de 10 à 20 jours, typiquement de 15 jours environ.

**12.** Utilisation selon l'une quelconque des revendications précédentes, **caractérisée en ce que** pour chaque application, le dispositif est maintenu sur la peau pendant une période de 6 à 48h environ, plus préférentiellement de 48 heures environ.

**13.** Utilisation selon l'une quelconque des revendications précédentes, **caractérisée en ce qu'**elle comprend trois applications successives au moins d'un dispositif, pendant une période de 48 heures environ, chaque application étant espacée de 15 jours environ.

## Patentansprüche

**1.** Verwendung einer Impfstoff-Formulierung zur Herstellung einer Zusammensetzung für die epikutane Impfung eines Säugetiers, vorzugsweise eines Menschen, **dadurch gekennzeichnet, dass** die Impfstoff-Formulierung in trockener Form und ohne Adjuvans vorliegt, und dass sie durch Applikation auf die intakte Haut des Säugetiers mithilfe einer Hautvorrichtung verabreicht wird, die bei Kontakt mit der Haut einen Kondensationsraum mit einem Volumen zwischen 1 und 300 $mm^3/cm^2$ Oberfläche der Vorrichtung bildet, wobei besagte Vorrichtung eine Impfstoff-Formulierung in einer Menge zwischen 0,1 µg und 500 µg pro $cm^2$ Oberfläche enthält, die Vorrichtung über einen Zeitraum von 6 bis 72 Stunden auf der Haut gehalten und besagte Applikation der Formulierung in trockener Form und ohne Adjuvans auf die intakte Haut eine für das Antigen spezifische Immunantwort auslöst, die durch die Produktion eines Antikörpers des Typs IgG gekennzeichnet ist.

**2.** Verwendung gemäß Anspruch 1, **dadurch gekennzeichnet, dass** die Wände des Kondensationsraums ein Material umfassen, das eine Permeabilitätsrate von kleiner als 10 $g/m^2/24$ h aufweist.

**3.** Verwendung gemäß einem der vorhergehenden Ansprüche, **dadurch gekennzeichnet, dass** der Kondensationsraum ein Volumen zwischen 2 und 100 $mm^3$ pro $cm^2$ Oberfläche besitzt.

**4.** Verwendung gemäß einem der vorhergehenden Ansprüche, **dadurch gekennzeichnet, dass** der Kondensationsraum eine Erhöhung des Wassergehaltes in dem Kondensationsraum und an der Epidermisoberfläche um 50% wenigstens eine Stunde nach Applikation induziert.

**5.** Verwendung gemäß einem der vorhergehenden Ansprüche, **dadurch gekennzeichnet, dass** die Vorrichtung mithilfe von Haftmitteln auf der Haut gehalten wird.

**6.** Verwendung gemäß einem der vorhergehenden Ansprüche, **dadurch gekennzeichnet, dass** die Impfstoff-Formulierung in Pulverform vorliegt.

**7.** Verwendung gemäß einem der vorhergehenden Ansprüche, **dadurch gekennzeichnet, dass** die Impfstoff-Formulierung auf einer Oberfläche der Vorrichtung durch Kräfte chemischer oder physikochemischer Art ohne Haftmaterialien auf dieser Oberfläche gehalten wird.

**8.** Verwendung gemäß einem der vorhergehenden Ansprüche, **dadurch gekennzeichnet, dass** die Impfstoff-Formulierung auf einer Oberfläche der Vorrichtung durch elektrostatische Kräfte oder Van-der-Waals-Kräfte gehalten wird.

**9.** Verwendung gemäß einem der vorhergehenden Ansprüche, **dadurch gekennzeichnet, dass** die Vorrichtung eine Impfstoff-Formulierung in einer Menge zwischen 20 µg und 300 µg pro $cm^2$ Oberfläche, bevorzugter zwischen 20 µg und 100 µg pro $cm^2$ Oberfläche, enthält.

**10.** Verwendung gemäß einem der vorhergehenden Ansprüche, **dadurch gekennzeichnet, dass** die Impfstoff-Formulierung wenigstens ein Antigen umfasst, das in Form eines Peptids oder Polypeptids oder als Polypeptid-Konjugate mit verschiedenartigen chemischen Komponenten vorliegt.

**11.** Verwendung gemäß einem der vorhergehenden Ansprüche, **dadurch gekennzeichnet, dass** sie die aufeinanderfolgende Applikation von wenigstens zwei Vorrichtungen, bevorzugt wenigstens drei Vorrichtungen, bei der Person umfasst, wobei jede Applikation in einem Abstand von ungefähr 7 bis 30 Tagen, bevorzugt von 10 bis 20 Tagen, typischerweise von ungefähr 15 Tagen, erfolgt.

**12.** Verwendung gemäß einem der vorhergehenden Ansprüche, **dadurch gekennzeichnet, dass** für jede Applikation die Vorrichtung über einen Zeitraum von ungefähr 6 bis 48 h, bevorzugter von ungefähr 48 h, auf der Haut gehalten wird.

**13.** Verwendung gemäß einem der vorhergehenden Ansprüche, **dadurch gekennzeichnet, dass** sie drei aufeinanderfolgende Applikationen wenigstens einer Vorrichtung über einen Zeitraum von ungefähr 48 Stunden umfasst, wobei jede Applikation in einem Abstand von ungefähr 15 Tagen erfolgt.

**Claims**

**1.** Use of a vaccine formulation for the preparation of a composition for the epicutaneous vaccination of a mammal, preferably a human, **characterized in that** the vaccine formulation is in dry form and devoid of adjuvant, and **in that** it is administered by application to the intact skin of the mammal by means of a cutaneous device forming in contact with the skin a condensation compartment having a volume ranging between 1 and 300 $mm^3/cm^2$ of surface of the device, said device containing a quantity of the vaccine formulation ranging between 0.1 $\mu$g and 500 $\mu$g per $cm^2$ of surface, the device being held onto the skin for a period of 6 to 72 hours, and said application of the formulation in dry form and devoid of adjuvant on intact skin inducing an antigen-specific immune response **characterized by** the production of IgG type antibodies.

**2.** Use according to claim 1, **characterized in that** the walls of the condensation compartment comprise a material having a moisture vapour transmission rate that is less than 10 $g/m^2/24h$.

**3.** Use according to any one of the preceding claims, **characterized in that** the condensation compartment has a volume ranging between 2 and 100 $nm^3$ per $cm^2$ of surface.

**4.** Use according to any one of the preceding claims, **characterized in that** the condensation compartment allows to induce an increased hydration rate in the condensation compartment and on the outer surface of the epidermis of at least 50% after one hour of application.

**5.** Use according to any one of the preceding claims, **characterized in that** the device is held onto the skin by adhesive means.

**6.** Use according to any one of the preceding claims, **characterized in that** the vaccine formulation is in powder form.

**7.** Use according to any one of the preceding claims, **characterized in that** the vaccine formulation is held on a surface of the device by forces of chemical or physicochemical type, in the absence of adhesive material on this surface.

**8.** Use according to any one of the preceding claims, **characterized in that** the vaccine formulation is held on a surface of the device by electrostatic forces or by Van der Waals forces.

**9.** Use according to any one of the preceding claims, **characterized in that** the device contains a quantity of vaccine formulation ranging between 20 $\mu$g and 300 $\mu$g per $cm^2$ of surface, more preferably between 20 $\mu$g and 100 $\mu$g per $cm^2$ of surface.

**10.** Use according to any one of the preceding claims, **characterized in that** the vaccine formulation comprises at least one antigen of peptide or polypeptide type or polypeptide conjugates with components of different chemical type.

**11.** Use according to any one of the preceding claims, **characterized in that** it comprises the successive application to the subject of at least two devices, preferably at least three, each application being spaced apart by about 7 to 30 days, preferably by 10 to 20 days, typically by about 15 days.

**12.** Use according to any one of the preceding claims **characterized in that**, for each application, the device is held onto the skin for a period of about 6 to 48 hours, more preferably for about 48 hours.

**13.** Use according to any one of the preceding claims, **characterized in that** it comprises at least three successive applications of a device, for a period of about 48 hours, each application being spaced apart by about 15 days.

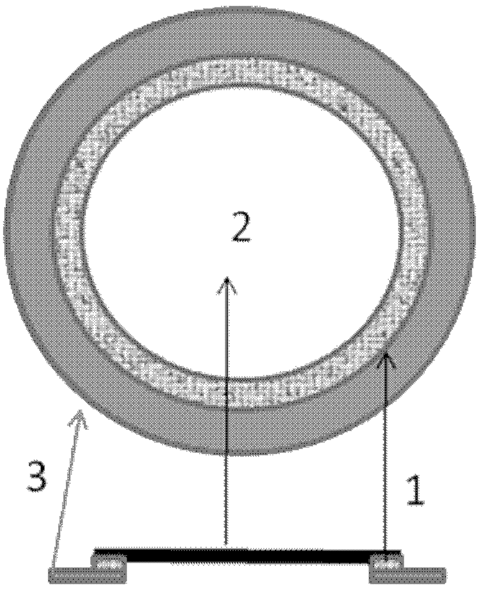


1A

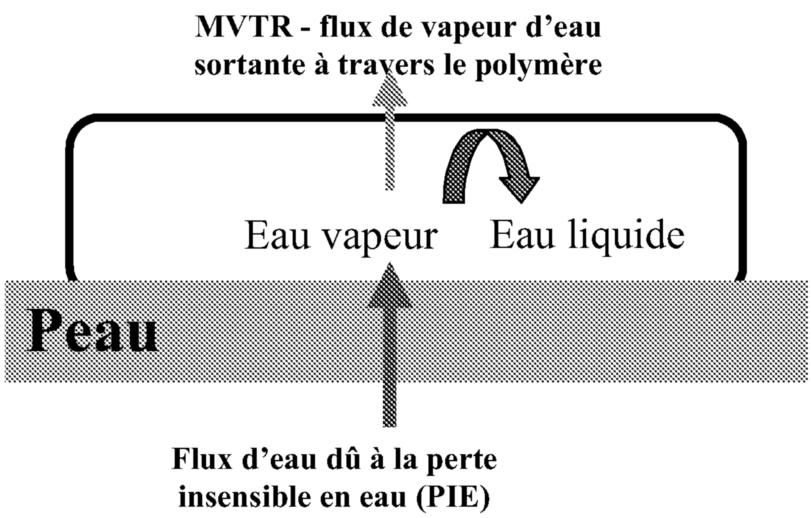


1B

**Figure 1**

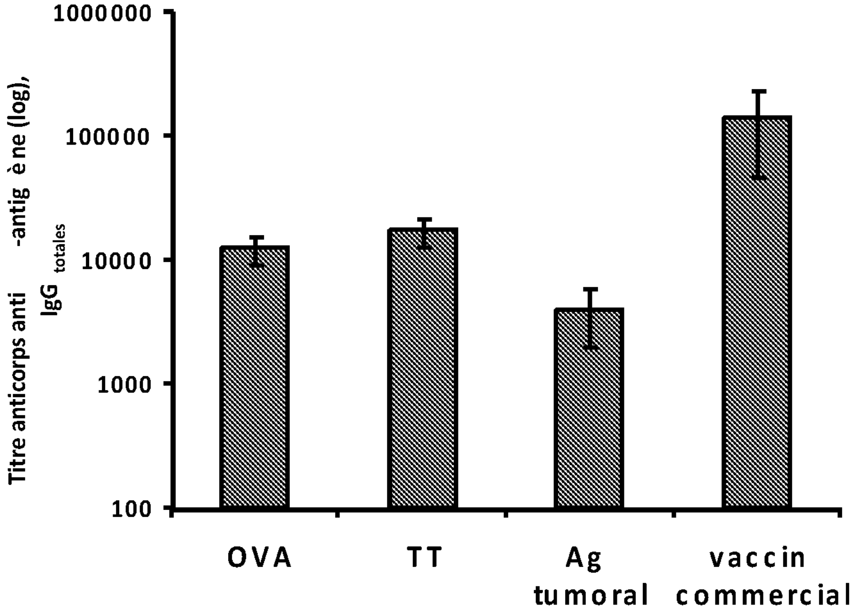


**Figure 2**

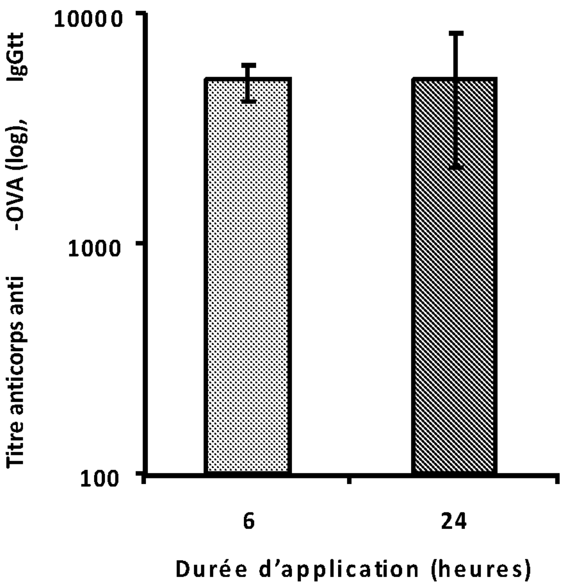


**Figure 3**

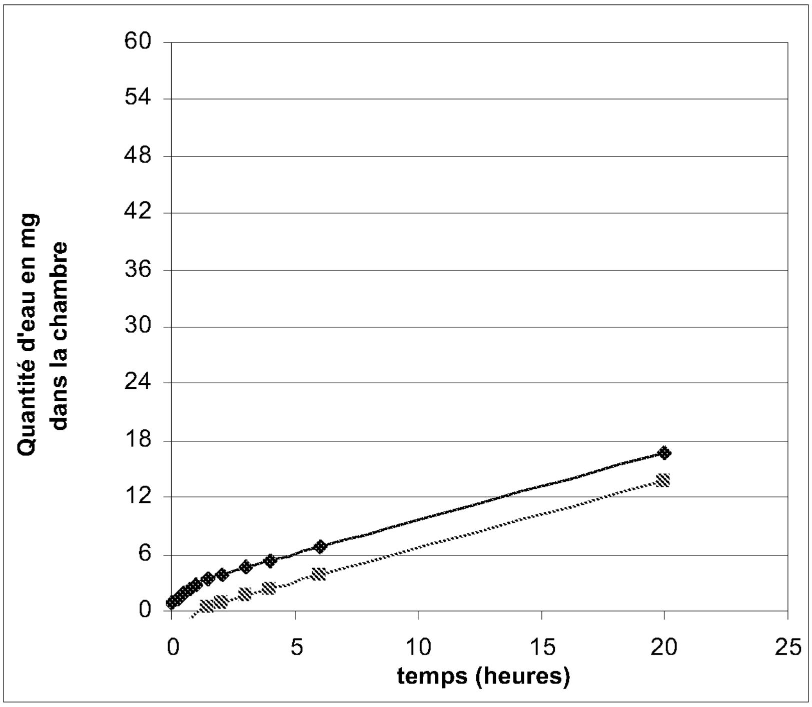


**Figure 4**

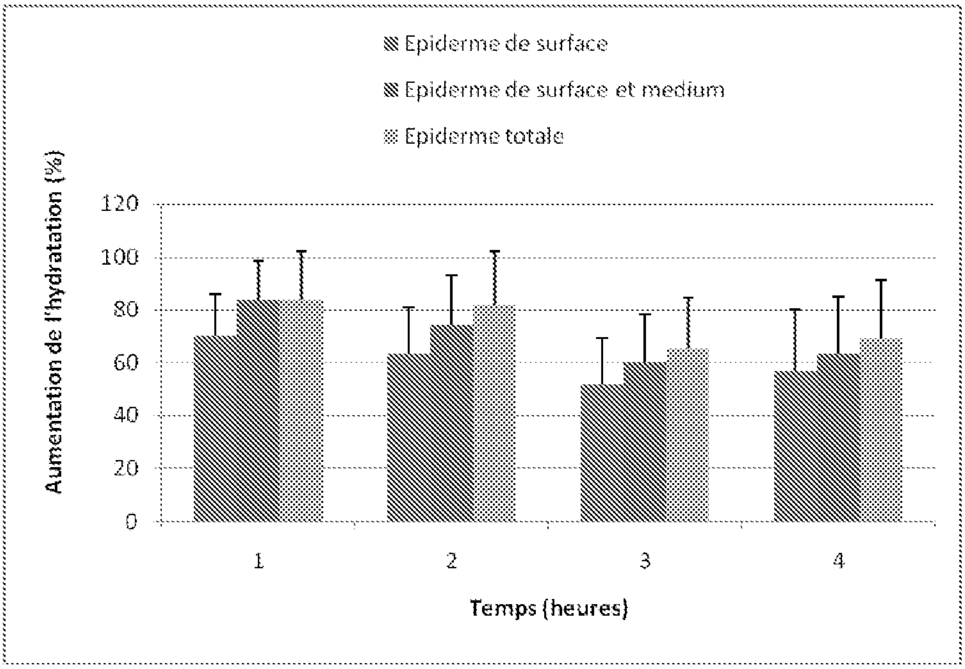


**Figure 5**

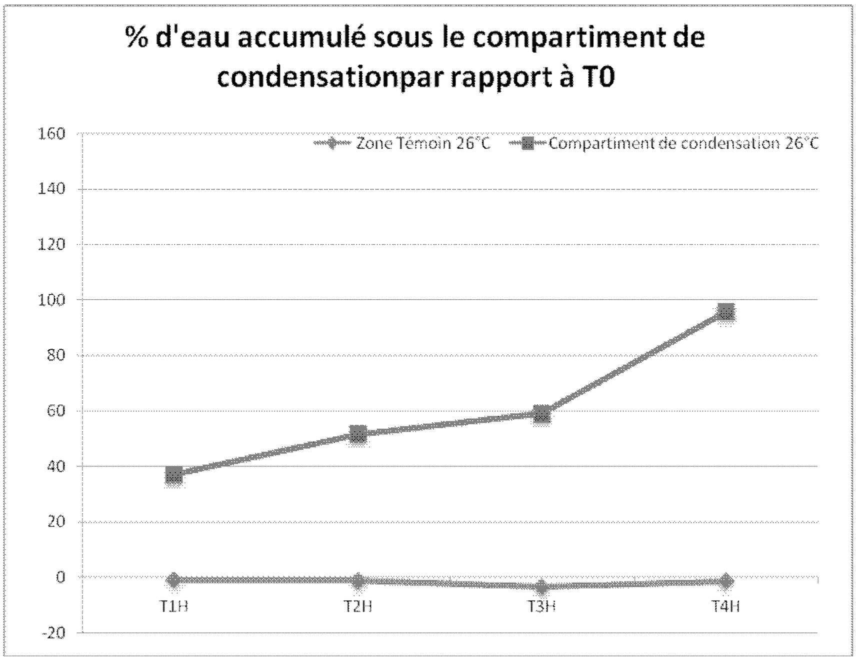


Figure 6

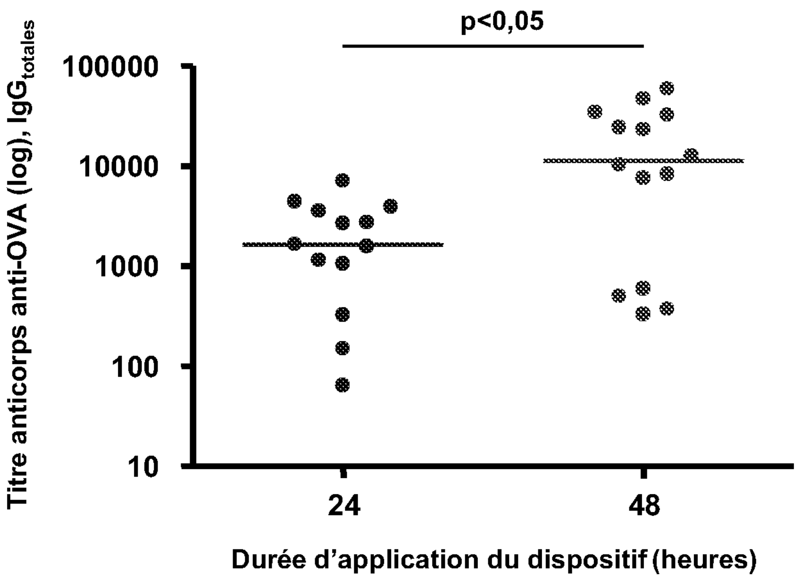


Figure 7A

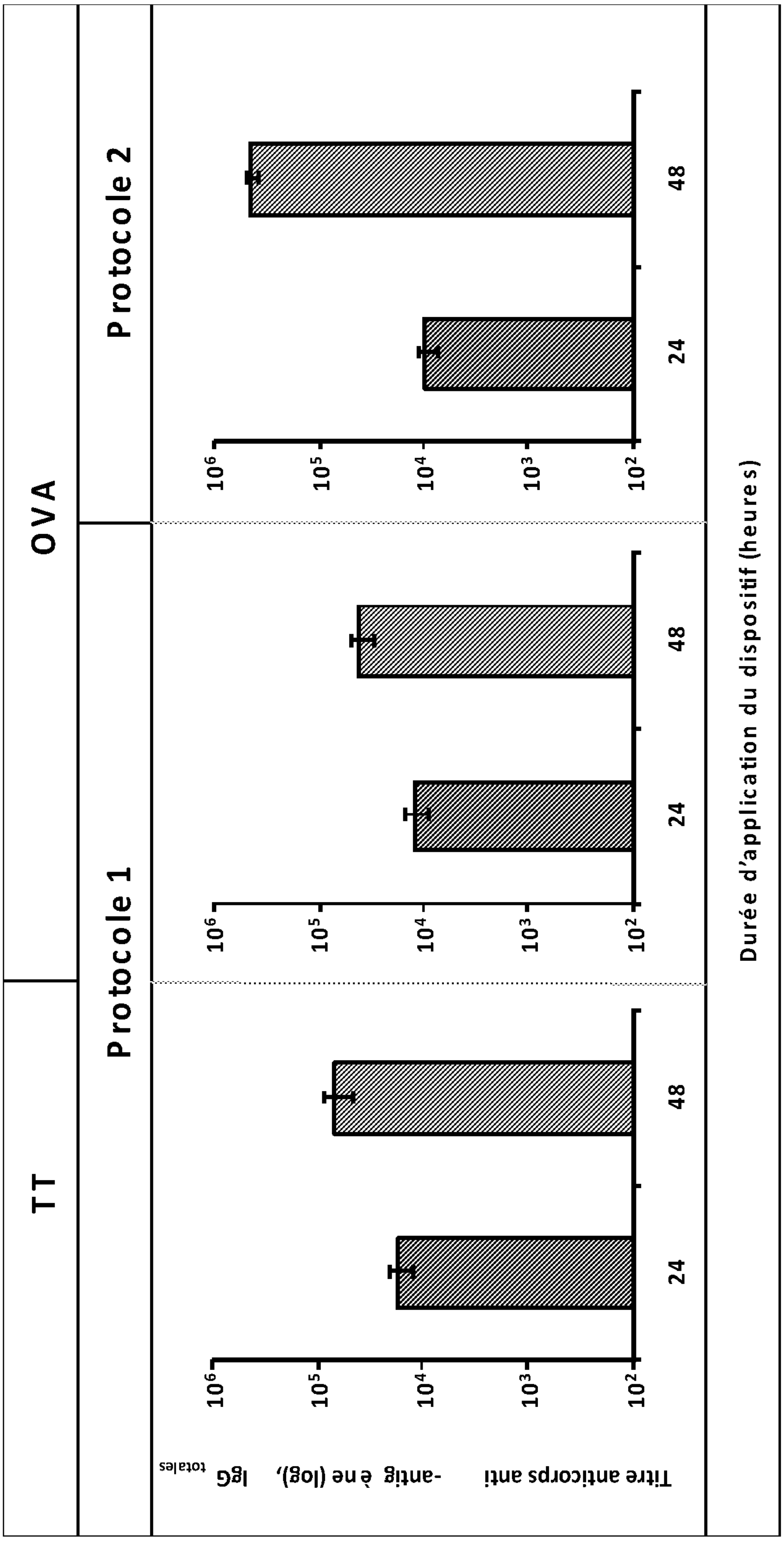
TT
OVA
Protocole 1
Protocole 2
Titre anticorps anti-antigène (log), IgG totales
$10^6$
$10^5$
$10^4$
$10^3$
$10^2$
24
48
Durée d'application du dispositif (heures)

Figure 7B

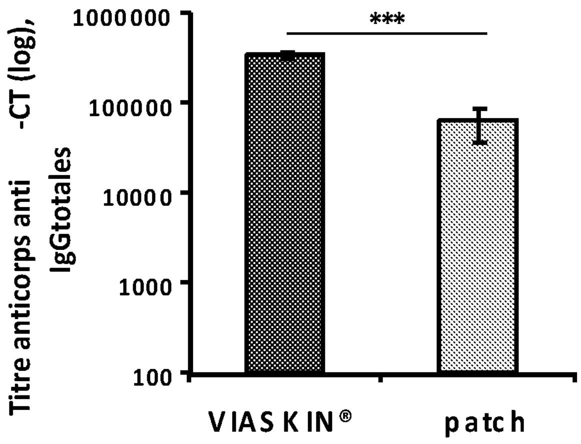


**Figure 8**

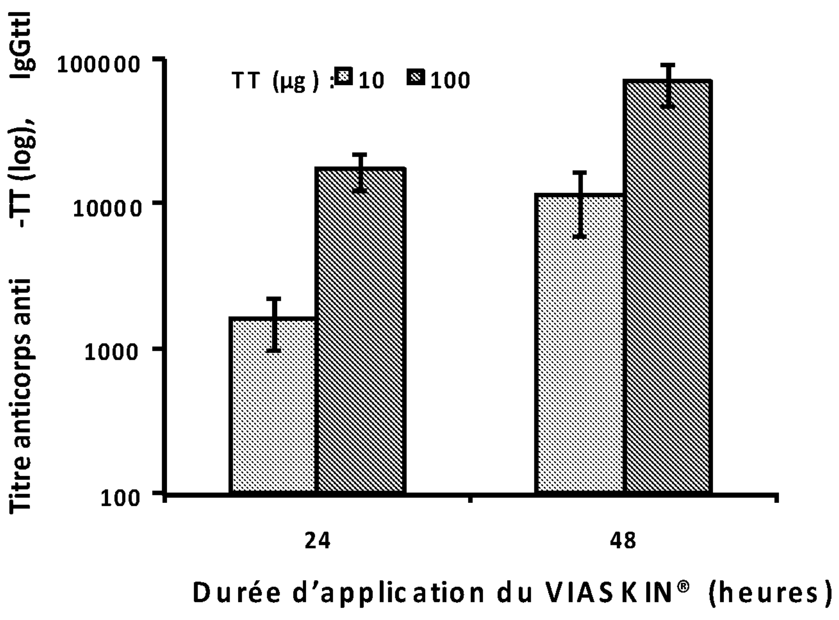


**Figure 9**

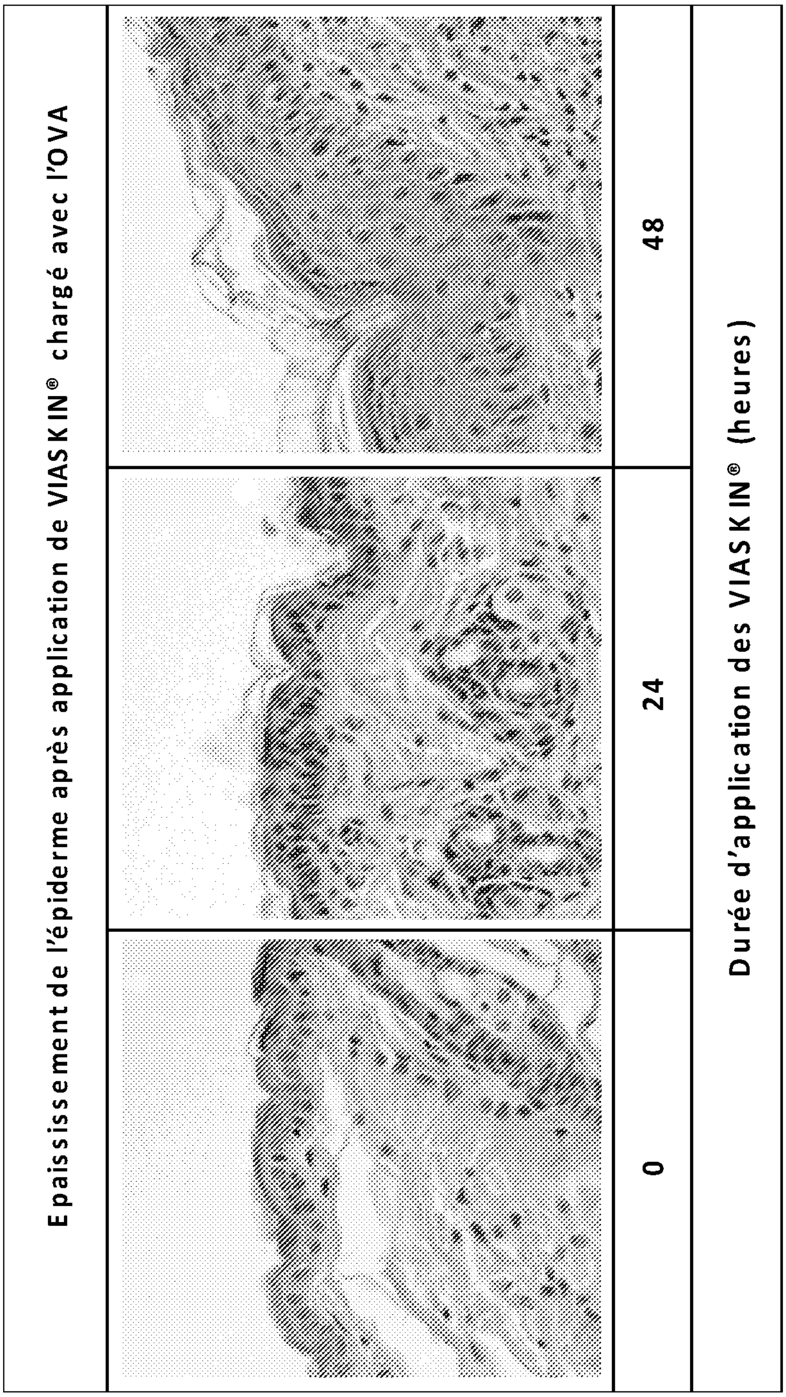
Epaississement de l'épiderme après application de VIASKIN® chargé avec l'OVA
0
24
48
Durée d'application des VIASKIN® (heures)

**Figure 10**

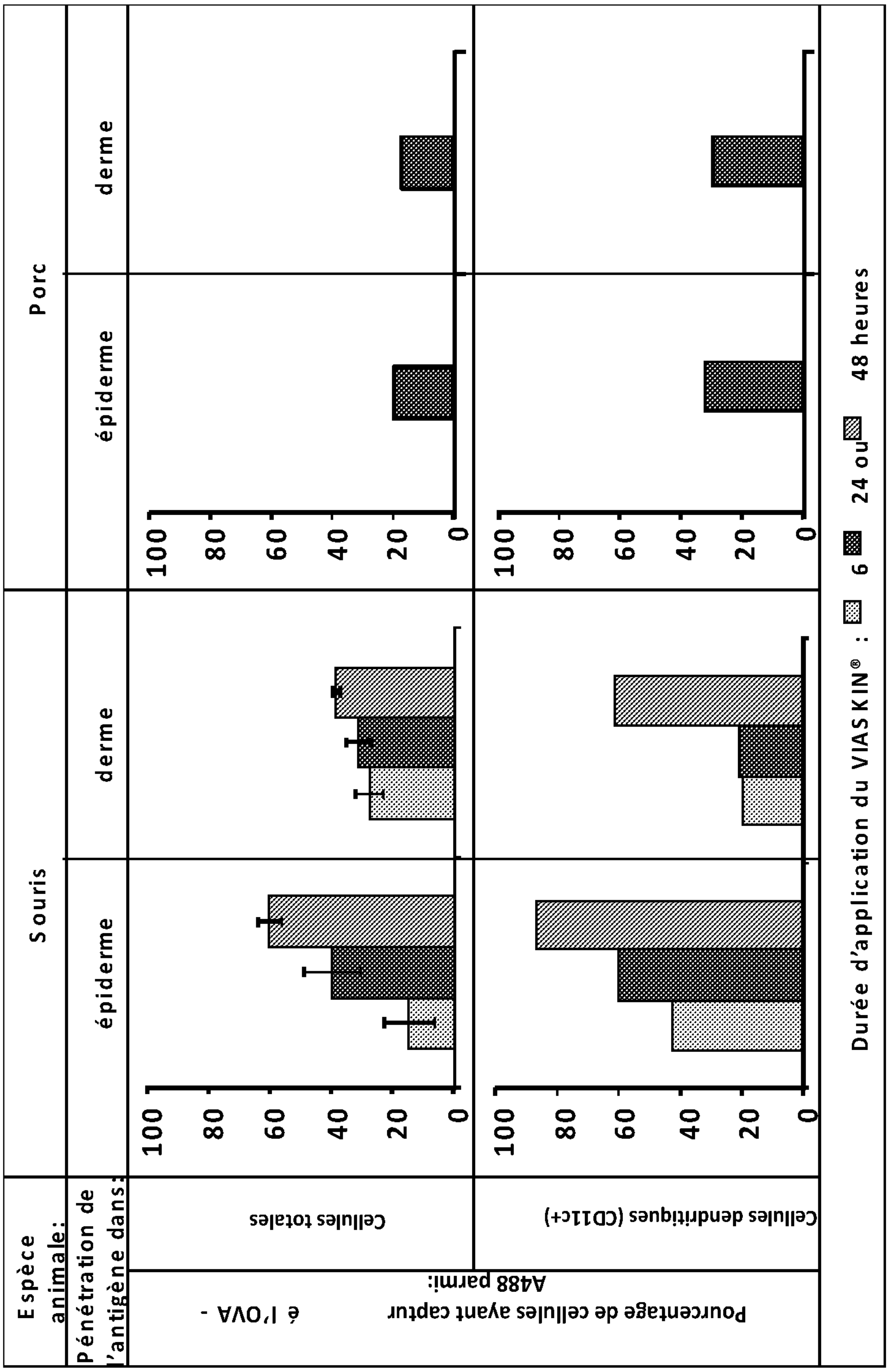
Espèce animale:
Souris
Porc
Pénétration de l'antigène dans:
épiderme
derme
épiderme
derme
Pourcentage de cellules ayant capturé l'OVA - A488 parmi:
Cellules totales
Cellules dendritiques (CD11c+)
100
80
60
40
20
0
Durée d'application du VIASKIN® : 6 24 ou 48 heures

**Figure 11**

Modification du réseau de cellules de Langherans après application de VIASKIN® chargé avec l'OVA l'OVA

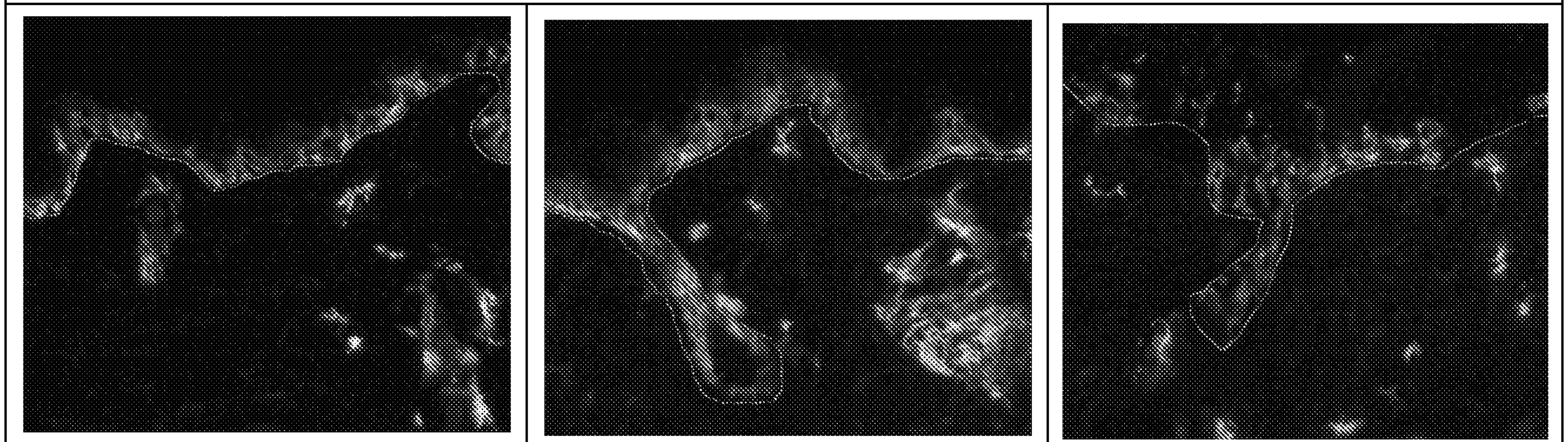

| 0 | 24 | 48 |
|---|---|---|

Durée d'application des VIASKIN® (heures)

**Figure 12**

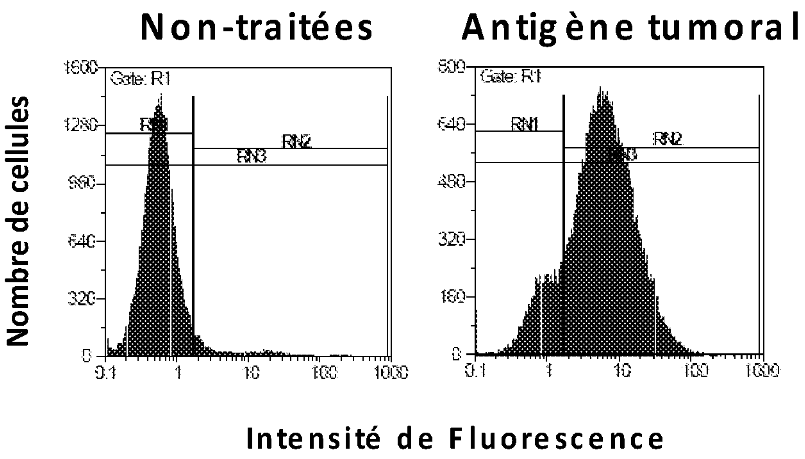


**Figure 13**

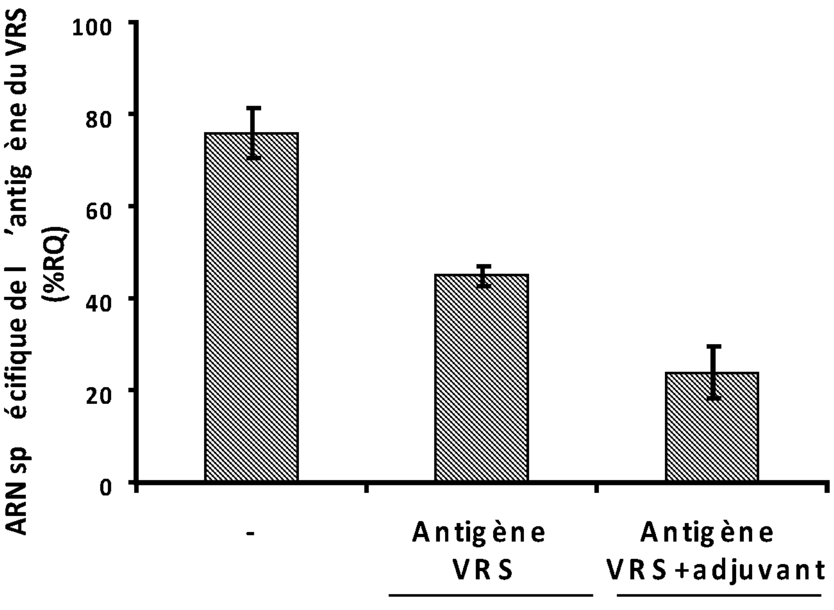


**Figure 14**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004030696 A
- WO 2007122226 A
- WO 02071950 A


### Littérature non-brevet citée dans la description


- **GLENN GM ; RAO M ; MATYAS GR ; ALVING CR.** Skin immunization made possible by cholera toxin. *Nature,* 1998, vol. 391, 851
- **GLENN GM ; SCHARTON-KERSTEN T ; VASSELL R ; MATYAS GR ; ALVING CR.** Transcutaneous immunization with bacterial ADP-ribosylating exotoxins as antigens and adjuvants. *Infect Immun.,* 1999, vol. 67, 1100-6
- **PAUL A ; CEVC G ; BACHHAWAT BK.** Transdermal immunization with large proteins by ultradeformable drug carriers. *Eur J Immunol.,* 1995, vol. 25 (12), 3521-4
- **GIRARD P. ; BERAUD A. ; SIRVENT A.** Study of three complementary techniques for measuring cutaneous hydration in vivo in human subjects: NMR spectroscopy, transient thermal transfer and corneometry - application to xerotic skin and cosmetics. *Skin Research and Technology,* 2000, vol. 6, 205-213